# EUROPÄISCHE PATENTSCHRIFT

(11) **EP 1 124 805 B1**
(45) Veröffentlichungstag und Bekanntmachung des Hinweises auf die Patenterteilung: **21.05.2003**
(21) Anmeldenummer: 99971416.5
(22) Anmeldetag: 29.10.1999
(51) Int. Cl.: C07D 239/50

(54) **VERFAHREN ZUR HERSTELLUNG VON 4-[(2',5'-DIAMINO-6'-HALOGENPYRIMIDIN-4'-YL)AMINO]-CYCLOPENT-2-ENYLMETHANOLEN**
METHOD FOR PRODUCING 4-[(2',5'- DIAMINO-6'- HALOPYRIMIDINE- 4'-YL)AMINO]- CYCLOPENT- 2-ENYLMETHANOLS
PROCEDE DE PRODUCTION DE 4-[(2',5'- DIAMINO-6'- HALOPYRIMIDIN- 4'-YL)AMINO]- CYCLOPENT-2- ENYLMETHANOLS

(30) Priorität: 30.10.1998 EP 98120529; 29.07.1999 US 146105 P
(43) Veröffentlichungstag der Anmeldung: 22.08.2001
(73) Patentinhaber: Lonza AG, 4052 Basel (CH)
(72) Erfinder: BRIEDEN, Walter, CH-3902 Brig-Glis (CH); SAIKALI, Elie, CH-3930 Visp (CH)
(74) Vertreter: Ritthaler, Wolfgang, Dr.
(86) Internationale Anmeldenummer: EP9908270
(87) Internationale Veröffentlichungsnummer: WO00026193

(56) Entgegenhaltungen:
- EP-A- 0 434 450
- EP-A- 0 684 236
- WO-A-91/01310
- WO-A-97/45529
- DE-A- 3 901 502
- M. LEGRAVEREND ET AL.: "A New Route to 2,5-Diamino-4,6-dichloropyrimidine, A Key Precursor of 9-Substituted Guanines" SYNTHESIS, Nr. 7, 1990, Seiten 587-9, XP002130733
- ANDERSEN M W ET AL: "The Synthesis of Modified D- and L-Anhydrohexitol Nucleosides" TETRAHEDRON LETTERS,NL,ELSEVIER SCIENCE PUBLISHERS, AMSTERDAM, Bd. 37, Nr. 45, 1996, Seiten 8147-50, XP004031067 ISSN: 0040-4039
- VINCE ET AL: "Synthesis and Anti-HIV Activity of Carbocyclic 2',3'-Didehydro-2',3'-dideoxy 2,6-Disubstituted Purine Nucleosides" JOURNAL OF MEDICINAL CHEMISTRY,US,AMERICAN CHEMICAL SOCIETY. WASHINGTON, Bd. 33, Nr. 1, 1990, Seiten 17-21, XP002109188 ISSN: 0022-2623
- EVANS C T ET AL: "POTENTIAL USE OF CARBOCYCLIC NUCLEOSIDES FOR THE TREATMENT OF AIDS CHEMO-ENZYMATIC SYNTHESES OF THE ENANTIOMERS OF CARBOVIR" JOURNAL OF THE CHEMICAL SOCIETY, PERKIN TRANSACTIONS 1,GB,CHEMICAL SOCIETY. LETCHWORTH, Nr. 5, 1992, Seiten 589-92, XP002089871 ISSN: 0300-922X in der Anmeldung erwähnt

## Beschreibung

Die vorliegende Erfindung betrifft ein neues Verfahren zur Herstellung von 4-[(2',5'-Diamino-6'-halogenpyrimidin-4'-yl)amino]cyclopent-2-enylmethanolen der allgemeinen Formel 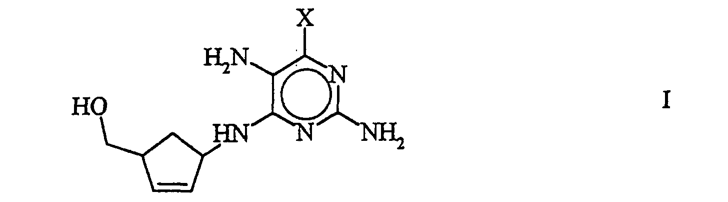 4-[(2',5'-Diamino-6'-halogenpyrimidin-4'-yl)amino]cyclopent-2-enylmethanole sind wichtige Zwischenprodukte zur Herstellung von antiviralen Nukleotidderivaten (WO 91/01310).

Bekannt ist die 3-stufige Synthese von 4-[(2',5'-Diamino-6'-chlorpyrimidin-4'yl)amino]cyclopent-2-enylmethanol ausgehend von 4-Acetamidocyclopent-2-enylmethanol durch Umsetzen mit 2-Amino-4,6-dichlorpyrimidin in Butanol mittels Diisopropylethylamin als Base. Dabei wird zunächst das [(2'-Amino-6'-chlorpyrimidin-4'-yl)amino]cyclopent-2-enylmethanol gebildet, welches dann in einer Folgestufe mittels Diazotierung in das entsprechende Amin überführt wird, das dann zum Endprodukt hydrolysiert wird (J. Chem. Soc. Perkin. Trans., 1, 1992, 589-592; EP-A-0 434 450). Dieses Verfahren hat den Nachteil, dass es zu kostspielig ist und das gewünschte Endprodukt nur in mässiger Ausbeute erhalten wird.

M. Legraverend et al. (Synthesis, 1990, 587-589) beschreiben die Herstellung von 3-(2,5-Diamino-6-chlorpyrimidin-4-yl)amino-5-(hydroxymethyl)cyclopentan-1,2-diol durch Umsetzung von 2,5-Diamino-4,6-dichlorpyrimidin mit 3-Amino-5-(hydroxymethyl)-cyclopentan-1,2-diol in Butanol. M. W. Andersen et al. (Tetrahedron Letters, 1996, 37, 8147-50) beschreiben die Herstellung von Anhydrohexitolnukleosiden.

Aufgabe der vorliegenden Erfindung ist es, ein 1-stufiges und damit kostengünstigeres Verfahren zur Herstellung von 4-[(2',5'-Diamino-6'-halogenpyrimidin-4'yl)amino]cyclopent-2-enylmethanolen zur Verfügung zu stellen, wobei die gewünschten Produkte in guter Ausbeute erhalten werden.

Diese Aufgabe wird mit dem Verfahren gemäss Anspruch 1 gelöst.

Überraschenderweise wurde gefunden, dass wenn man anstatt 2-Amino-4,6-dichlorpyrimidin als Ausgangsmaterial ein 2,5-Diamino-4,6-dihalogenpyrimidin der allgemeinen Formel 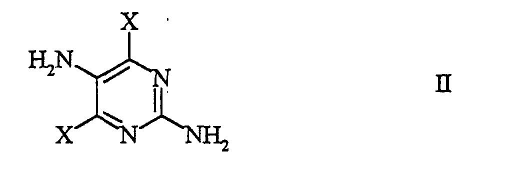 verwendet und dieses in Gegenwart eines Alkali- oder Erdalkalimetallcarbonats oder eines Alkali- oder Erdalkalimetallhydrogencarbonats als Base in einem polar protischen Lösungsmittel mit einem 4-Aminocyclopent-2-enylmethanol der Formel 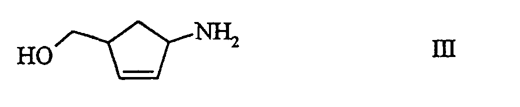 oder einem Salz davon reagieren lässt, das gewünschte Endprodukt der allgemeinen Formel 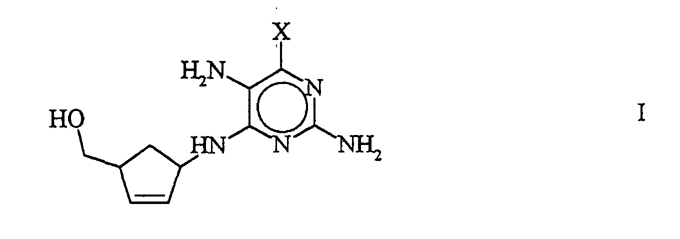 um ein Vielfaches kostengünstiger und in guter Ausbeute erhalten wird.

Der Substituent X bedeutet ein Halogenatom wie F, Cl, Br oder I.

Die 2,5-Diamino-4,6-dihalogenpyrimidine wie das 2,5-Diamino-4,6-dichlorpyrimidin können gemäss der EP-A-0 684 236 hergestellt werden.

Als 4-Aminocyclopent-2-enylmethanole können sowohl die racemischen als auch die optisch aktiven Verbindungen wie die (1R,4S)-, (1S,4R)-, (1R,4R)-, oder (1S,4S)-4-Aminocyclopent-2-enylmethanole eingesetzt werden. Geeignete Salze davon sind Säureadditionssalze, insbesondere die Hydrohalogenidsalze, beispielsweise die Hydrochloride oder Hydrobromide. Die 4-Aminocyclopent-2-enylmethanole, insbesondere das (1R,4S)- oder das (1S,4R)-Enantiomere, können gemäss der WO 97/45529 hergestellt werden.

Die Umsetzung wird in Gegenwart eines Alkali- oder Erdalkalimetallcarbonats oder eines Alkali- oder Erdalkalimetallhydrogencarbonats als Base durchgeführt. Als Alkalimetallcarbonat bzw. Alkalimetallhydrogencarbonat können Natrium- und Kaliumcarbonat oder Natrium- oder Kaliumhydrogencarbonat eingesetzt werden. Als Erdalkalimetallcarbonat bzw. Erdalkalimetallhydrogencarbonat können Calcium- oder Magnesiumcarbonat oder Calciumhydrogencarbonat eingesetzt werden. Vorzugsweise wird die Umsetzung in Gegenwart eines Alkalimetallhydrogencarbonats wie Natriumhydrogencarbonat durchgeführt.

Die Base wird zweckmässig im Überschuss bezogen auf das 2,5-Diamino-4,6-dihalogenpyrimidin eingesetzt, vorzugsweise werden 1 bis 4 mol Base pro mol 2,5-Diamino-4,6-dihalogenpyrimidin eingesetzt.

Als polar protische Lösungsmittel sind insbesondere C₁₋₄-Alkohole wie Methanol, Ethanol, Propanol ISOPZOPYLALKOHOL und Butanol und seine Isomere geeignet.

Zweckmässig wird die Umsetzung bei einer Temperatur von 20 °C bis zur Rückflusstemperatur des entsprechenden Lösungsmittels, vorzugsweise von 50 °C bis zur Rückflusstemperatur, durchgeführt. Zweckmässig werden das 4-Aminocyclopent-2-enylmethanol und das 2,5-Diamino-4,6-dihalogenpyrimidin äquimolar eingesetzt.

Nach einer üblichen Umsetzungszeit von 2 bis 20 h können dann die Endprodukte gemäss Formel I, vorzugsweise das (1S,4R)-4-[(2',5'-Diamino-6'-halogenpyrimidin-4'-yl)-amino]cyclopent-2-enylmethanol, durch übliche Aufarbeitungsmethoden erhalten werden.

### Beispiel

### Herstellung von 4-[(2',5'-Diamino-6'-chlorpyrimidin-4'-yl)amino]cyclopent-2-enylmethanol in Gegenwart von Natriumhydrogencarbonat

(1S,4R)-4-Aminocyclopent-2-enylmethanolhydrochlorid (0,14 mol, 23,25 g), Ethanol (3 mol, 138,12 g, 176 ml), 2,5-Diamino-4,6-dichlorpyrimidin (0,14 mol, 25 g) und Natriumhydrogencarbonat (0,34 mol, 28,68 g) wurden in einen trockenen Reaktor gegeben. Diese Mischung wurde bis zur Rückflusstemperatur (ca. 80 °C) 16 h lang erhitzt. Die Umsatzrate wurde gemäss TLC mit 13/1 Methylenchlorid : Methanol als Laufinittel getestet. Die Reaktionsmischung wurde auf Raumtemperatur abgekühlt und 45 min lang gerührt. Die Salze wurden durch Filtration entfernt und der Filterkuchen 2-MAL mit Ethanol (0,86 mol, 39,5 g, 50 ml) gewaschen.

Nachdem 2/3 der organischen Phase durch Vakuumdestillation entfernt waren, wurde tropfenweise Hexan (150 ml) hinzugefügt. Die Suspension wurde auf unter 10 °C gekühlt. Nach Filtration wurde im Vakuum bei 50 °C getrocknet.

Es wurden 21,5 g (0,08 mol) Endprodukt, entsprechend einer Ausbeute von 60 %, erhalten.

## Patentansprüche

1. Verfahren zur Herstellung von 4-[(2',5'-Diamino-6'-halogenpyrimidin-4'yl)amino]cyclopent-2-enylmethanolen der allgemeinen Formel 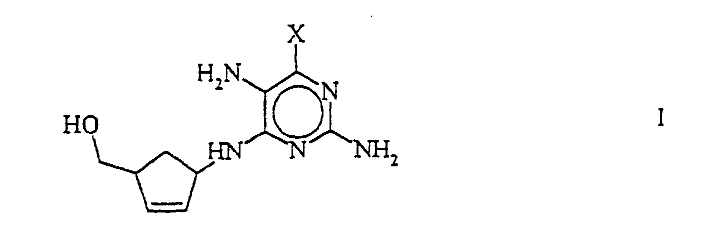 worin X ein Halogenatom bedeutet, **dadurch gekennzeichnet, dass** man ein 2,5-Diamino-4,6-dihalogenpyrimidin der allgemeinen Formel 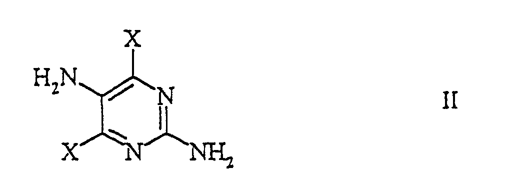 worin X die genannte Bedeutung hat, mit einem 4-Aminocyclopent-2-enylmethanol, der Formel 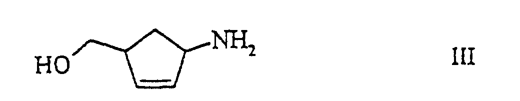 oder einem seiner Salze, in Gegenwart eines Alkali- oder Erdalkalimetallcarbonats oder in Gegenwart eines Alkali- oder. Erdalkalimetallhydrogencarbonats in einem polar protischen Lösungsmittel zum Endprodukt der allgemeinen Formel I umsetzt.

2. Verfahren nach Anspruch 1, **dadurch gekennzeichnet, dass** als polar protisches Lösungsmittel ein C₁₋₄-Alkohol eingesetzt wird

3. Verfahren nach Anspruch 1 oder 2, **dadurch gekennzeichnet, dass** die Umsetzung bei einer Temperatur von 20 °C bis zur Rückflusstemperatur des entsprechenden Lösungsmittels durchgeführt wird.

## Claims

1. Process for preparing 4-[(2',5'-diamino-6'-halopyrimidin-4'-yl)amino)cyclopent-2-enylmethanols of the general formula 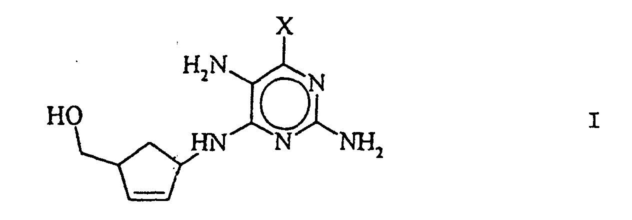 in which X is a halogen atom, **characterized in that** a 2,5-diamino-4,6-dihalopyrimidine of the general formula 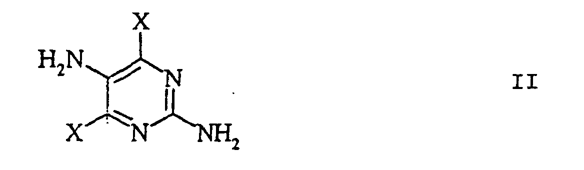 in which X is defined as above, is reacted with a 4-aminocyclopent-2-enylmethanol of the formula 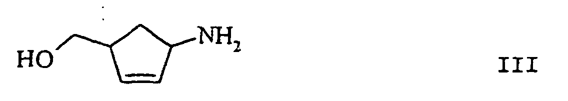 or one of its salts, in the presence of an alkali metal carbonate or alkali earth metal carbonate or in the presence of an alkali metal bicarbonate or alkali earth metal bicarbonate, in a polar protic solvent to give the end product of the general formula I.

2. Process according to Claim 1, **characterized in that** the polar protic solvent used is a C₁₋₄-alcohol.

3. Process according to Claims 1 or 2, **characterized in that** the reaction is carried out at a temperature of from 20°C to the reflux temperature of the solvent in question.

## Revendications

1. Procédé de production de 4-[(2',5'-diamino-6'-halogénopyrimidin-4'-yl)amino]cyclopent-2-énylméthanols de formule générale 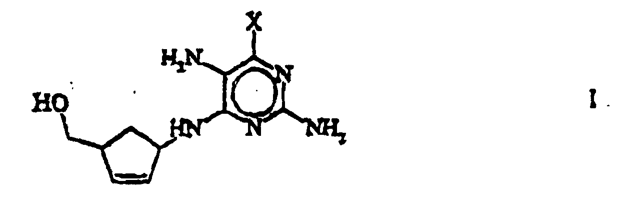 où X représente un atome d'halogène, **caractérisé en ce que** l'on convertit ' une 2,5-diamino-4,6-dihalogéno-pyrimidine de formule générale 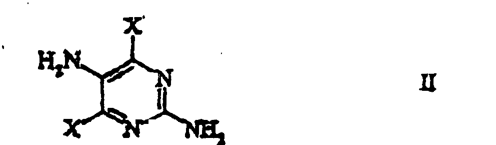 où X a la signification citée, avec un 4-aminocyclopent-2-énylméthanol de formule 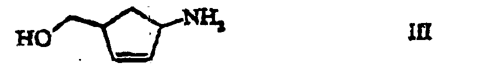 ou l'un de ses sels, en présence d'un carbonate de métal alcalin ou alcalino-terreux ou en présence d'un hydrogénocarbonate de métal alcalin ou alcalino-terreux dans un solvant protique polaire, en le produit final de formule générale I.

2. Procédé selon la revendication 1 **caractérisé en ce que** l'on utilise un alcool en C₁₋₄ comme solvant protique polaire.

3. Procédé selon la revendication 1 ou 2 **caractérisé en ce que** la conversion est conduite à une température de 20°C à la température de reflux du solvant correspondant.
